# EUROPÄISCHE PATENTSCHRIFT

(11) **EP 3 432 730 B1**
(45) Veröffentlichungstag und Bekanntmachung des Hinweises auf die Patenterteilung: **08.11.2023**
(21) Anmeldenummer: 17708464.7
(22) Anmeldetag: 01.03.2017
(51) Int. Cl.: A23K 20/10, A23K 20/163, A23K 50/00, A23L 29/206, A23L 29/219, A23L 29/256, A23L 29/281, A23L 3/3463, A23L 3/3472, A23L 33/155, A23P 10/30, A61K 9/10, A61K 9/16, A23L 33/15

(54) **MEHRPHASIGE WIRKSTOFFZUBEREITUNGEN**
MULTIPHASE ACTIVE SUBSTANCE PREPARATIONS
PRÉPARATIONS DE PRINCIPES ACTIFS MULTIPHASES

(30) Priorität: 24.03.2016 EP 16162118
(43) Veröffentlichungstag der Anmeldung: 30.01.2019
(73) Patentinhaber: BASF SE, 67056 Ludwigshafen am Rhein (DE)
(72) Erfinder: HANSEN, Rene, 2750 Ballerup (DK); KORTEGAARD, Katrine, 2750 Ballerup (DK); KRYLBO, Inge-Lise, 2860 Söborg (DK); HARZ, Hans-Peter, 67373 Dudenhofen (DE); PELLETIER, Wolf, 76879 Ottersheim (DE); BRUHNS, Stefan, 68305 Mannheim (DE); JORGENSEN, Mona Glenstrup, 2750 Ballerup (DK)
(74) Vertreter: BASF IP Association
(86) Internationale Anmeldenummer: PCT/EP2017/054723
(87) Internationale Veröffentlichungsnummer: WO 2017/162413

(56) Entgegenhaltungen:
- EP-A1- 2 721 933
- EP-A2- 0 065 193
- EP-A2- 0 239 086
- WO-A1-01/51088
- WO-A1-01/51088
- WO-A1-2005/013708
- US-A1- 2008 317 822
- US-A1- 2008 317 822

## Beschreibung

Die vorliegende Erfindung betrifft feste mindestens dreiphasige Wirkstoffzubereitungen, in denen zwei separate Phasen multipartikulär in eine kohärente wirkstoff- und antioxidansfreie Phase eingebettet sind und eine der eingebetteten Phasen antioxidationsfrei und mindestens einen schwer wasserlöslichen oxidationsempfindlichen Wirkstoff und die Zweite wirkstofffrei und mindestens ein schwer wasserlösliches Antioxidans enthält. Die erfindungsgemäßen Wirkstoffzubereitungen weisen eine gegenüber dem Stand der Technik verbesserte Lagerstabilität auf.

Es ist bekannt, dass bestimmte Wirkstoffe oxidationsempfindlich sind und Stabilitätsprobleme bei der Lagerung zeigen. Unter oxidationsempfindlich soll erfindungsgemäß die Fähigkeit des Wirkstoffs verstanden werden, mit einer oxidierenden Substanz unter Normalbedingungen (d.h. zwischen +5° C und +40° C bei 1013,25 hPa) zu reagieren. Zu den Oxidationsreaktionen sollen dabei die Übertragung von Sauerstoff auf den Wirkstoff, die Abstraktion von Wasserstoff und die Abgabe von Elektronen, optional mit der Abgabe von Protonen, gehören. Zur Erhöhung der Stabilität des Wirkstoffs gegen oxidativen Abbau ist es vorteilhaft Antioxidantien den Wirkstoffzubereitungen zuzusetzen. Normalerweise werden Antioxidantien und Wirkstoffe dabei gemeinsam vorgemischt und anschließend den übrigen Bestandteilen hinzugefügt.

EP 0065193 offenbart ein Verfahren zur Herstellung von feinverteilten, pulverförmigen Carotinoid- bzw. Retinoidpräparaten. Hieraus ist bekannt, dass Antioxidantien bei der Herstellung von Wirkstoffzubereitungen entweder der wässrigen oder der wirkstoffhaltigen Lösungsmittelphase zugesetzt werden können, sie jedoch vorzugweise gemeinsam mit dem Wirkstoff und optional tensidartigen Stabilisatoren in der Lösungsmittelphase vorzumischen sind.

Dieser überraschende Befund der hervorragenden Stabilisierung wird in der Ausbildung separater multipartikulärer entweder antioxidans- oder wirkstoffenthaltender Phasen vermutet, die in der kohärenten Phase eingebettet vorliegen und die erfindungsgemäße Wirkstoffzubereitung kennzeichnen. Erfindungsgemäß wird dabei unter dem Begriff "separat" räumlich getrennt voneinander verstanden
Bei dem in der kohärenten Phase eingebetteten oxidationsempfindlichen Wirkstoff, handelt es sich bevorzugt um einen schwer wasserlöslichen, oxidationsempfindlichen Wirkstoff. "Schwer wasserlöslich" bedeutet im Sinne der Erfindung, dass diese bei Standardbedingungen (Temperatur 25° C, Luftdruck 1013,25 hPa) eine Löslichkeit von kleiner 0,1 Gew.-% in hochgereinigtem Wasser gemäß der Vorgaben des Europäischen Arzneibuches Ph.Eur. 8.0 aufweist.

Insbesondere ist die erste in die kohärente Phase eingebettete Phase, die einen schwer wasserlöslichen, oxidationsempfindlichen Wirkstoff enthält, antioxidansfrei und die zweite in die kohärente Phase eingebettete Phase, die ein Antioxidans enthält, wirkstofffrei.

Antioxidansfrei bedeutet im Sinne der Erfindung, dass die einen schwer wasserlöslichen, oxidationsempfindlichen Wirkstoff enthaltende Phase im Durchschnitt weniger als 1000ppm, bevorzugt weniger als 100ppm und besonders bevorzugt weniger als 10ppm Antioxidans enthält.

Vice versa bedeutet wirkstofffrei im Sinne der Erfindung, dass die ein Antioxidans enthaltende Phase im Durchschnitt weniger als 1000ppm, bevorzugt weniger als 100ppm und besonders bevorzugt weniger als 10ppm schwer wasserlöslichen, oxidationsempfindlichen Wirkstoff enthält.

Unter den schwer wasserlöslichen, oxidationsempfindlichen Wirkstoffen der Erfindung sind besonders Retinoide und/oder Vitamin D bevorzugt.

Unter Retinoiden sind im Rahmen der vorliegenden Erfindung Vitamin A Alkohol (Retinol) und seine Derivate wie Vitamin A Aldehyd (Retinal), Vitamin A Säure (Retinsäure) und Vitamin A Ester (z .B. Retinylacetat, Retinylpropionat, Retinylstearat und Retinylpalmitat) zu verstehen. Der Begriff Retinoide umfasst die all-trans und cis-Verbindungen, bevorzugt die all-trans und geometrischen cis-Verbindungen und besonders bevorzugt die all-trans Verbindungen.

Unter Vitamin D sind erfindungsgemäß Vitamin D₃ und Vitamin D₂ und ihre biologisch aktiven Metabolite, wie 1-α-Hydroxy-Vitamin D₃ und 25-Hydroxy-Viamin D₃ zu verstehen.

Ganz besonders bevorzugt sind hierbei erfindungsgemäß Retinylacetat, Retinylpalmitat und/oder Vitamin D₃.

In der kohärenten Phase enthält die erfindungsgemäße Wirkstoffzubereitung zumindest ein Schutzkolloid, welches ausgewählt ist aus Hydrokolloiden pflanzlichen und/oder tierischen Ursprungs. Diese Hydrokolloide können physikalisch und/oder chemisch modifiziert sein.

Bevorzugt sind die Schutzkolloide ausgewählt aus der Gruppe der Pflanzengummis, modifizierten Pflanzengummis, Gelatine, modifizierten Gelatine, modifizierten Stärke, Ligninsulfonate, Chitosane, Carrageenane, Caseine, Caseinate, Molkeproteine, Zeine, modifizierter Cellulosen, Pectine, modifizierten Pectine, Pflanzenproteine und modifizierten Pflanzenproteine oder Mischungen davon.

Unter Pflanzengummis sind dabei erfindungsgemäß zu verstehen Agar, Alginsäure, Alginat, Chicle, Dammar, Eibisch Extrakte, Gellan, Guarkernmehl, Gummi Arabicum, Ghatti-Gummi, Gummi aus Wegerichkernspelze, Gummi aus Fichtensaft, Gummi aus Lärchensaft, Johannisbrotkernmehl, Karaya, Konjakmehl, Mastix, Tarakernmehl, Traganth oder Xanthan.

Zu den Gelatinen gehört erfindungsgemäß Schweinegelatine, Rindergelatine, Fischgelatine oder Geflügelgelatine, jeweils vom Typ A und B und in einem Bloombereich von 0 bis 300.

Unter Pflanzenproteinen sind erfindungsgemäß zu verstehen Erbsenprotein, Bohnenprotein, Kartoffelprotein, Lupinenprotein, Sojaprotein, Getreideprotein oder Erdnussprotein.

Besonders bevorzugt in der kohärenten Phase sind erfindungsgemäß Gelatine, modifizierte Stärke und/oder Pflanzengummis.

Ganz besonders bevorzugt sind erfindungsgemäß Gelatine, modifizierte Stärke und/oder Gummi Arabicum in der kohärenten Phase und hier insbesondere Wirkstoffzubereitungen, die Gelatine, modifizierte Stärke und/oder Gummi Arabicum in der kohärenten Phase, in Kombination mit den schwer wasserlöslichen, oxidationsempfindlichen Wirkstoffen Retinylacetat, Retinylpalmitat und/oder Vitamin D₃ in der Wirkstoffphase enthalten.

Die erfindungsgemäße Wirkstoffzubereitung enthält zudem eine in die kohärente Phase eingebettete Antioxidans-enthaltende Phase. Das Antioxidans ist dabei ausgewählt aus der Gruppe bestehend aus Tocopherol, Butylhydroxytoluol (BHT), Butylhydroxyanisol (BHA), Tert-Butylhydroxychinolin (TBHQ), Ethoxyquin (EQ), Carnosol, Carnosolsäure, Ascorbylpalmitat und Ascorbylstearat oder Mischungen davon. Unter Tocopherol wird erfindungsgemäß dl*-α*-Tocopherol, d-*α*-Tocopherol, *β*-Tocopherol, *γ*-Tocopherol und/oder *δ*-Tocopherol verstanden.

Bevorzugt sind von den genannten die schwer wasserlöslichen Antioxidantien. "Schwer wasserlöslich" bedeutet im Sinne der Erfindung, dass diese bei Standardbedingungen (Temperatur 25° C, Luftdruck 1013,25 hPa) eine Löslichkeit von kleiner 0,1 Gew.-% in hochgereinigtem Wasser gemäß der Vorgaben des Europäischen Arzneibuches Ph.Eur. 8.0 aufweisen.

Besonders bevorzugt sind erfindungsgemäß die schwer wasserlöslichen Antioxidantien Butylhydroxytoluol (BHT) und/oder Tocopherol und hier insbesondere Wirkstoffzubereitungen, die Butylhydroxytoluol (BHT) und/oder Tocopherol in der Antioxidans-enthaltenden Phase, in Kombination mit den Schutzkolloiden Gelatine, modifizierte Stärke und/oder Gummi Arabicum in der kohärenten Phase und in Kombination mit den oxidationsempfindlichen Wirkstoffen Retinylacetat, Retinylpalmitat und/oder Vitamin D₃ in der Wirkstoffphase enthalten.

Der Anteil der schwer wasserlöslichen Antioxidantien an der Wirkstoffzubereitung liegt bei 0,1 bis 20 Gew.-%, bevorzugt bei 0,5 bis 15 Gew.-%. Besonders bevorzugt sind ein Anteil von 0,5 bis 10 Gew.-% Tocopherol und/oder 2 bis 15 Gew.-% Butylhydroxytoluol (BHT) an der Wirkstoffzubereitung.

Der Anteil der oxidationsempfindlichen Wirkstoffe an der Wirkstoffzubereitung liegt bei 0,1 bis 60 Gew.-%. Handelt es sich bei den oxidationsempfindlichen Wirkstoffen um Vitamin D und/oder ein Retinoid ist/sind ein Anteil von 0,1 bis 5 Gew.-% Vitamin D und/oder 10 bis 60 Gew.-% Retinoid bevorzugt, besonders bevorzugt ist ein Anteil von 0,1 bis 1 Gew.-% Vitamin D und/oder 15 bis 40 Gew.-% Retinoid. Bei Vitamin D handelt es sich dabei insbesondere um Vitamin D₃ und bei Retinoid insbesondere um Retinylpalmitat und/oder Retinylacetat.

Der Anteil der Schutzkolloide an der Wirkstoffzubereitung liegt bei 1 bis 80 Gew.-%, bevorzugt bei 10 bis 70 Gew.-%. Besonders bevorzugt handelt es sich bei den Schutzkolloiden dabei um Gelatine, modifizierte Stärke und/oder Pflanzengummis.

Alle Angaben zur prozentualen Zusammensetzung der erfindungsgemäßen Wirkstoffzubereitung sind Massenanteile bezogen auf deren Trockenmasse, wobei die Summe der Prozentangaben der Komponenten 100% ergibt.

Zur Verbesserung der Herstellung und der Anwendungseigenschaften der Wirkstoffzubereitung ist es gegebenenfalls zweckmäßig, weitere Komponenten als Hilfs- und Zusatzstoffe zuzusetzen. Bevorzugte Hilfs- und Zusatzstoffe sind Weichmacher, Emulgatoren, Öle, wasserlösliche Salze und/oder Trennmittel.

Zur Anpassung der mechanischen Stabilität der kohärenten Phase der Wirkstoffzubereitung kann es zweckmäßig sein, dem Schutzkolloid zumindest einen Weichmacher zuzusetzen, wie Polyole, Zucker oder Zuckeralkohole, z.B. Saccharose, Glukose, Glukosesirup, Stärkehydrolysate, Fruktose, Fruktosesirup, Lactose, Maltose, Xylose, Arabinose, Ribose, Trehalose, Invertzucker, Sorbit, Mannit, Manitol, Dextrin, Maltodextrin, Glycerin, Polyetherglykole oder Isomalt. Die Bezeichnung Isomalt steht für einen Zuckeraustauschstoff, der auch unter dem Markennamen Palatinit^{®} (Fa. Südzucker, Deutschland) geführt wird. Isomalt ist eine hydrierte Isomaltulose, die aus etwa gleichen Teilen 6-O-α-D-Glucopyranosyl-D-sorbit und 1-O-α-D-Glucopyranosyl-D-mannit besteht. Bevorzugt verwendete Weichmacher sind Saccharose, Glucosesirup und Lactose.

Weiterhin können Emulgatoren zur Stabilisierung der Phasen bei der Herstellung der erfindungsgemäßen Wirkstoffzubereitung eingesetzt werden, beispielsweise Mono- und Diglyceride, Monoglycerin-Fettsäureester, Polyglycerin-Fettsäureester, SorbitanFettsäureester, Propylenglycol-Fettsäureester, Monoglycerin-Zitronensäureester, Zucker-Fettsäureester oder Lecithin. Bevorzugt verwendete Emulgatoren sind Mono- und Diglyceride, Monoglycerin-Fettsäureester und Lecithin.

Unter Umständen kann es auch vorteilhaft sein, zusätzlich ein physiologisch zugelassenes Öl tierischen oder pflanzlichen Ursprungs zu verwenden, wie beispielsweise Sesamöl, Maiskeimöl, Baumwollsaatöl, Sojabohnenöl, Erdnußöl, Sonnenblumenöl, Rapsöl, Kokosöl, Palmöl, Olivenöl, Distelöl, tierische Fette, Schmalz, Talg, durch Hydrieren, Fraktionieren oder Umesterung modifizierte Öle oder Mischungen, um darin mindestens einen schwer wasserlöslichen, oxidationsempfindlichen Wirkstoff und/oder ein schwer wasserlösliches Antioxidans in der optimalen Konzentration zu lösen. Bevorzugte Öle sind Maiskeimöl, Sonnenblumenöl und Rapsöl.

Zusätzlich können in die kohärente Phase der Wirkstoffzubereitung vorteilhafterweise noch wasserlösliche anorganische und/oder organische Salze zugefügt werden, wie beispielsweise Natriumascorbat, Kaliumascorbat, Calciumascorbat, Natriumerythorbat, Kaliumerythorbat, Natriumbenzoat, Kaliumbenzoat, Natriumcitrat, Kaliumcitrat, Alkaliphosphate, Alkaliacetate, Alkaliphytate und Mischungen davon. Bevorzugte Salze sind Natriumascorbat, Natriumbenzoat, Natriumcitrat und Dinatriumhydrogenphosphat.

Um unerwünschtes Verklumpen zu vermeiden und die Fließfähigkeit zu verbessern, werden zweckmäßigerweise schwer wasserlösliche, feinpartikuläre Trennmittel mit mittleren Partikelgrößen kleiner 15µm (x50,3 nach DIN ISO 9276-2) zugegeben, die an der Oberfläche der pulverförmigen Wirkstoffzubereitung anlagern. Das bevorzugte Trennmittel ist dabei ausgewählt aus der Gruppe bestehend aus Siliziumdioxid, hydrophob modifizierte Kieselsäure, Tricalciumphosphat (TCP), Calciumkarbonat, Natriumkarbonat, Kaliumkarbonat, Magnesiumkarbonat, Calciumoxid, Magnesiumoxid, Dicalciumdiphosphat, Calciumsilikat, Magnesiumsilikat, Magnesiumtrisilikat, Natrium-Aluminiumsilikat, Talkum, Kaolin, Calciumstearat, Magnesiumstearat, Stärken der verschiedenen botanischen Quellen, Cellulose oder Mischungen davon. Besonders bevorzugt sind dabei Siliziumdioxid, Tricalciumphosphat (TCP), hydrophob modifizierte Kieselsäure und Maisstärke.

Der Anteil an Hilfs- und Zusatzstoffen ist erfindungsgemäß 0,1 bis 70 Gew.-%, bevorzugt 1 bis 50 Gew.-% und besonders bevorzugt 5 bis 30 Gew.-% bezogen auf die Trockenmasse der erfindungsgemäßen Wirkstoffzubereitung, wobei die Summe der Prozentangaben der Komponenten 100% ergibt.

Mit dem Begriff Dispersion sind im Rahmen der vorliegenden Erfindung sowohl Emulsionen als auch Suspensionen, bevorzugt Emulsionen und besonders bevorzugt Ölin-Wasser-Emulsionen gemeint.

Die Herstellung der erfindungsgemäßen Dispersionen erfolgt in Apparaturen, die durch Eintrag mechanischer Energie für die Feinverteilung der schwer wasserlöslichen Komponenten in wässrigen Lösungen sorgen. Der Energieeintrag liegt dabei bei 10⁶ bis 10⁹ J/m³ bezogen auf das Dispersionsvolumen. Die Herstellung kann sowohl in einem kontinuierlichen als auch einem diskontinuierlichen Prozess erfolgen. Geeignete Apparaturen hierfür sind mit Rotor-Stator-Systemen ausgerüstete Emulgierkessel, Mühlen wie Kolloidmühlen und Kugelmühlen, Homogenisatoren wie Hochdruckhomogenisatoren, Turbinenhomogenisatoren und Zahnkranzdispergiermaschinen oder Kombinationen dieser Apparaturen.

Bei erfindungsgemäßer Ausführung sind die schwer wasserlöslichen Komponenten in der Dispersion fein verteilt. Als Maß für die Feinverteilung dient die Messung der mittleren Partikelgröße (x50,3 nach DIN ISO 9276-2) der schwer wasserlöslichen Komponenten in Dispersion mittels Laserdifraktometrie nach ISO 13320-1, beispielsweise mittels eines Mastersizer 3000 der Firma Malvern Instruments Ltd. Bevorzugt sind mittlere Partikelgrößen von kleiner 5µm, besonders bevorzugt kleiner 1µm.

Aus der Dispersion kann das Umwandeln in ein pulverförmiges Präparat in an sich bekannter Weise erfolgen, z.B. durch Granulieren, Pastillieren, Prillen, Schuppen, Compoundieren oder formgebendes Trocknen. Das so hergestellte pulverförmige Präparat hat einen, über Siebanalyse ermittelten, mittleren Partikelgröße (x50, 3 nach DIN ISO 9276-2) kleiner 5 mm, bevorzugt kleiner 1 mm und besonders bevorzugt kleiner 0,5 mm.

Mikroskopische Aufnahmen der Wirkstoffzubereitungen unter Kontrastierung der jeweiligen Phasen sind schematisch in den folgenden Abbildungen zur Illustration der Erfindung dargestellt.

Abbildung A: erfindungsgemäße Wirkstoffzubereitung gemäß der Beispiele 1-4A mit kohärenter Phase (I), in die separat voneinander und multipartikulär sowohl eine wirkstoffhaltige Phase (II) als auch eine antioxidanshaltige Phase (III) eingebettet sind.

Abbildung B: nicht erfindungsgemäße Wirkstoffzubereitung gemäß der Beispiele 1-4B mit kohärenter Phase (I), in die multipartikulär eine Mischphase (IV) eingebettet ist, die aus einer gemeinsamen Mischung von Wirkstoff und Antioxidans besteht.

Abbildung C: nicht erfindungsgemäße Wirkstoffzubereitung gemäß der Beispiele 1-4C mit nicht durchgehend kohärenter Phase (V), in die eine undefinierte Mischphase (VI) von Wirkstoff und Antioxidans eingebettet ist.

Im Folgenden werden die Herstellung der erfindungsgemäßen Wirkstoffzubereitungen, die Prüfung der Lagerstabilität und die Anwendung beispielhaft näher erläutert.

### Erfindungsgemäßes Beispiel 1A

Eine Gelatine-Lösung wurde in einem beheizbaren Emulgierkessel bei 70° C hergestellt, indem 900 g Gelatine (Typ A, 100 Bloom) in 2500 g Wasser für 30 Minuten durch Quellen in Lösung gebracht wurde. Dieser Gelatine-Lösung wurden 250 g eines Glucose-Sirups und 70 g Na₂HPO₄ zugesetzt, um eine Schutzkolloid-Lösung zu erhalten. Dieser Schutzkolloid-Lösung wurden 180 g BHT unter starkem Rühren* zugesetzt. Dann wurden 800 g geschmolzenen Vitamin-A-Acetats zugegeben und eine Emulsion unter starkem Rühren* hergestellt. Die so hergestellte Emulsion wurde über eine Düse in einem Sprühturm sprühgetrocknet, in dem hydrophobe Kieselsäure (x50,3~10pm) bei 60° C fluidisiert wurde. Die noch feuchten Partikel wurden für 30 min bei 60° C Lufteintrittstemperatur im darunterliegenden Wirbelbett nachgetrocknet. Die so hergestellte Wirkstoffzubereitung wurde mit 1A bezeichnet. Die mittlere Partikelgröße in Dispersion war x50,3~0,36µm, die mittlere Partikelgröße des pulverförmigen Präparates war x50,3~230µm und die Restfeuchte des pulverförmigen Präparates 2,9%.

### Nicht-erfindungsgemäßes Beispiel 1B

Eine Gelatine-Lösung wurde in einem beheizbaren Emulgierkessel bei 70° C hergestellt, indem 900 g Gelatine (Typ A, 100 Bloom) in 2500 g Wasser für 30 Minuten durch Quellen in Lösung gebracht wurden. Dieser Gelatine-Lösung wurden 250 g eines Glucose-Sirups und 70 g Na₂HPO₄ zugesetzt, um eine Schutzkolloid-Lösung zu erhalten. 180 g BHT und 800 g geschmolzenen Vitamin-A-Acetat wurden separat gemischt und diese Mischung wurde der Schutzkolloid-Lösung unter Rühren zugesetzt. Nach der Zugabe wurde eine Emulsion unter starkem Rühren* hergestellt. Die so hergestellte Emulsion wurde über eine Düse in einem Sprühturm sprühgetrocknet, in dem hydrophobe Kieselsäure (x50,3~10pm) bei 60° C fluidisiert wurde. Die noch feuchten Partikel wurden für 30 min bei 60° C Lufteintrittstemperatur im darunterliegenden Wirbelbett getrocknet. Die so hergestellte Wirkstoffzubereitung wurde mit 1B bezeichnet. Die mittlere Partikelgröße in Dispersion war x50,3~0,35µm, die mittlere Partikelgröße des pulverförmigen Präparates war x50,3~240µm und die Restfeuchte des pulverförmigen Präparates 2,6%.

### Nicht-erfindungsgemäßes Beispiel 1C

Eine Gelatine-Lösung wurde in einem beheizbaren Emulgierkessel bei 70° C hergestellt, indem 900 g Gelatine (Typ A, 100 Bloom) in 2500 g Wasser für 30 Minuten durch Quellen in Lösung gebracht wurden. Dieser Gelatine-Lösung wurden 250 g eines Glucose-Sirups und 70 g Na2HPO4 zugesetzt, um eine Schutzkolloid-Lösung zu erhalten. Dieser Schutzkolloid-Lösung wurden 800 g geschmolzenen Vitamin-A-Acetats unter starkem Rühren* zugesetzt. Dann wurden 180 g BHT zugegeben und eine Emulsion unter starkem Rühren* hergestellt. Die so hergestellte Emulsion wurde über eine Düse in einem Sprühturm sprühgetrocknet, in dem hydrophobe Kieselsäure (x50,3~10pm) bei 60° C fluidisiert wurde. Die noch feuchten Partikel wurden für 30 min bei 60° C Lufteintrittstemperatur im darunterliegenden Wirbelbett nachgetrocknet. Die so hergestellte Wirkstoffzubereitung wurde mit 1C bezeichnet. Die mittlere Partikelgröße in Dispersion war x50,3~5,2µm, die mittlere Partikelgröße des pulverförmigen Präparates war x50,3~220µm und die Restfeuchte des pulverförmigen Präparates 2,6%.

### Erfindungsgemäßes Beispiel 2A

Eine Gummi-Arabicum-Lösung wurde in einem beheizbaren Emulgierkessel bei 70° C hergestellt, indem 1100 g Gummi Arabicum in 2500 g Wasser für 30 Minuten durch Quellen in Lösung gebracht wurden. Dieser Gummi-Arabicum-Lösung wurden 300 g Saccharose und 20 g Na-Ascorbat zugesetzt, um eine Schutzkolloid-Lösung zu erhalten. Dieser Schutzkolloid-Lösung wurden 70 g DL-α-Tocopherol und 1 g Ascorbylpalmitat unter starkem Rühren* zugesetzt. Dann wurden 400 g Vitamin-A-Palmitat zugegeben und eine Emulsion unter starkem Rühren* hergestellt. Die so hergestellte Emulsion wurde über eine Düse in einem Sprühturm bei 120° C Lufteintrittstemperatur und 90° C Luftaustrittstemperatur sprühgetrocknet. Die noch feuchten Partikel wurden für 30 min bei 60° C Lufteintrittstemperatur im darunterliegenden Wirbelbett nachgetrocknet. Die so hergestellte Wirkstoffzubereitung wurde mit 2A bezeichnet. Die mittlere Partikelgröße in Dispersion war x50,3~0,63µm, die mittlere Partikelgröße des pulverförmigen Präparates war x50,3~140µm und die Restfeuchte des pulverförmigen Präparates 3,3%.

### Nicht-erfindungsgemäßes Beispiel 2B

Eine Gummi-Arabicum-Lösung wurde in einem beheizbaren Emulgierkessel bei 70° C hergestellt, indem 1100 g Gummi Arabicum in 2500 g Wasser für 30 Minuten durch Quellen in Lösung gebracht wurden. Dieser Gummi-Arabicum-Lösung wurden 300 g Saccharose und 20 g Na-Ascorbat zugesetzt, um eine Schutzkolloid-Lösung zu erhalten. 70 g DL-α-Tocopherol 1 g Ascorbylpalmitat und 400 g Vitamin-A-Palmitat wurden separat gemischt und diese Mischung wurde der Schutzkolloid-Lösung unter Rühren zugesetzt. Nach der Zugabe wurde eine Emulsion unter starkem Rühren* hergestellt. Die so hergestellte Emulsion wurde über eine Düse in einem Sprühturm bei 120° C Lufteintrittstemperatur und 90° C Luftaustrittstemperatur sprühgetrocknet. Die noch feuchten Partikel wurden für 30 min bei 60° C Lufteintrittstemperatur im darunterliegenden Wirbelbett nachgetrocknet. Die so hergestellte Wirkstoffzubereitung wurde mit 2B bezeichnet. Die mittlere Partikelgröße in Dispersion war x50,3~0,64µm, die mittlere Partikelgröße des pulverförmigen Präparates war x50,3~180µm und die Restfeuchte des pulverförmigen Präparates 3,2%.

### Nicht-erfindungsgemäßes Beispiel 2C

Eine Gummi-Arabicum-Lösung wurde in einem beheizbaren Emulgierkessel bei 70° C hergestellt, indem 1100 g Gummi Arabicum in 2500 g Wasser für 30 Minuten durch Quellen in Lösung gebracht wurden. Dieser Gummi-Arabicum-Lösung wurden 300 g Saccharose und 20 g Na-Ascorbat zugesetzt, um eine Schutzkolloid-Lösung zu erhalten. Dieser Schutzkolloid-Lösung wurden 400 g Vitamin-A-Palmitat unter starkem Rühren* zugesetzt. Dann wurden 70 g DL-α-Tocopherol und 1 g Ascorbylpalmitat zugegeben und eine Emulsion unter starkem Rühren* hergestellt. Die so hergestellte Emulsion wurde über eine Düse in einem Sprühturm bei 120° C Lufteintrittstemperatur und 90° C Luftaustrittstemperatur sprühgetrocknet. Die noch feuchten Partikel wurden für 30 min bei 60° C Lufteintrittstemperatur im darunterliegenden Wirbelbett nachgetrocknet. Die so hergestellte Wirkstoffzubereitung wurde mit 2C bezeichnet. Die mittlere Partikelgröße in Dispersion war x50,3~7,6µm, die mittlere Partikelgröße des pulverförmigen Präparates war x50,3~170pm und die Restfeuchte des pulverförmigen Präparates 3,3%.

### Stabilitätsprüfung für Lagerung von Wirkstoffzubereitungen mit Vitamin A im Prämix

Die Stabilität der so hergestellten Wirkstoffzubereitungen wurde in einem Stress-Prämixtest geprüft. Dafür wurden Prüfmuster von jeweils 100 mg der hergestellten Wirkstoffzubereitung und 4 g Prämixmischung in 50ml-Glasfläschen eingewogen. Die Prämixmischung bestand aus 60% Weizengrießkleie, 30% 50%-igem auf Kieselsäure geträgertem Cholinchlorid und 10% Spurenelementmischung. Die Spurenelementmischung bestand aus 46% FeSO₄x7H₂O, 38% CuSO₄x5H₂O, 12% ZnO und 4% MnO. Nach Zugabe aller Inhaltsstoffe wurden die Prüfmuster sorgfältig von Hand gemischt. Diese Prüfmuster wurden in einer Klimakammer bei 40° C und 70% für 4 Wochen gelagert. Vor Beginn der Lagerung und nach Abschluss der Lagerung wurde der Vitamin-A-Gehalt der Prüfmuster bestimmt. Der Vitamin-A-Gehalt wurde nach Verordnung (EG) Nr. 152/2009, Anhang IV, Teil A bestimmt. Aus dem Verhältnis der Vitamin-A-Gehalte nach und vor der Lagerung wurde die Retention berechnet.

Die Retentionswerte der Beispiele sind in der folgenden Tabelle zusammengefasst:

| Bezeichnung | Retention (%) |
|---|---|
| 1A | 70 |
| 1B | 64 |
| 1C | 62 |
| 2A | 49 |
| 2B | 33 |
| 2C | 29 |

Je höher die Retention desto besser die Stabilität der Wirkstoffzubereitung im Prämix. Vergleicht man die Stabilität der Wirkstoffzubereitung aus den jeweiligen erfindungsgemäßen Bespielen mit den dazugehörigen nicht-erfindungsgemäßen Beispielen (1A mit 1B und 1C oder 2A mit 2B und 2C), so erkennt man deutlich die Stabilitätsverbesserung.

### Erfindungsgemäßes Beispiel 3A

Eine Gelatine-Lösung wurde in einem beheizbaren Emulgierkessel bei 70° C hergestellt, indem 900 g Gelatine (Typ A, 240 Bloom) in 2500 g Wasser für 30 Minuten durch Quellen in Lösung gebracht wurden. Dieser Gelatine-Lösung wurden 250 g Saccharose zugesetzt, um eine Schutzkolloid-Lösung zu erhalten. Dieser Schutzkolloid-Lösung wurden 60 g Covi-Ox T 70 EU (Gemisch aus D-*α*-Tocopherol, *β*-Tocopherol, *γ*-Tocopherol und *δ*- Tocopherol mit Sojaöl von BASF SE) unter starkem Rühren* zugesetzt. Dann wurde eine Vormischung aus 100 g Sonnenblumenöl und 4 g Vitamin D₃ zugegeben und eine Emulsion unter starkem Rühren* hergestellt. Die so hergestellte Emulsion wurde über eine Düse in einem Sprühturm sprühgekühlt, in dem Maisstärke (x50,3~5pm) bei 15° C fluidisiert wurde. Die noch feuchten Partikel wurden für 30 min bei 60° C Lufteintrittstemperatur im darunterliegenden Wirbelbett nachgetrocknet. Die so hergestellte Wirkstoffzubereitung wurde mit 3A bezeichnet. Die mittlere Partikelgröße in Dispersion war x50,3~0,34µm, die mittlere Partikelgröße des pulverförmigen Präparates war x50,3~250µm und die Restfeuchte des pulverförmigen Präparates 2,7%.

### Nicht-erfindungsgemäßes Beispiel 3B

Eine Gelatine-Lösung wurde in einem beheizbaren Emulgierkessel bei 70° C hergestellt, indem 900 g Gelatine (Typ A, 240 Bloom) in 2500 g Wasser für 30 Minuten durch Quellen in Lösung gebracht wurden. Dieser Gelatine-Lösung wurden 250 g Saccharose zugesetzt, um eine Schutzkolloid-Lösung zu erhalten.

60 g Covi-Ox T 70 EU (Gemisch aus D-*α*-Tocopherol, *β*-Tocopherol, *γ*-Tocopherol und *δ*-Tocopherol mit Sojaöl von BASF SE), 100 g Sonnenblumenöl und 4 g Vitamin D₃ wurden separat gemischt und diese Mischung wurde der Schutzkolloid-Lösung unter starkem Rühren zugesetzt. Nach der Zugabe wurde eine Emulsion unter starkem Rühren* hergestellt. Die so hergestellte Emulsion wurde über eine Düse in einem Sprühturm sprühgekühlt, in dem Maisstärke (x50,3~5pm) bei 15° C fluidisiert wurde. Die noch feuchten Partikel wurden für 30 min bei 60° C Lufteintrittstemperatur im darunterliegenden Wirbelbett nachgetrocknet. Die so hergestellte Wirkstoffzubereitung wurde mit 3B bezeichnet. Die mittlere Partikelgröße in Dispersion war x50,3~0,37µm, die mittlere Partikelgröße des pulverförmigen Präparates war x50,3~240µm und die Restfeuchte des pulverförmigen Präparates 3,2%.

### Nicht-erfindungsgemäßes Beispiel 3C

Eine Gelatine-Lösung wurde in einem beheizbaren Emulgierkessel bei 70° C hergestellt, indem 900 g Gelatine (Typ A, 240 Bloom) in 2500 g Wasser für 30 Minuten durch Quellen in Lösung gebracht wurden. Dieser Gelatine-Lösung wurden 250 g Saccharose zugesetzt, um eine Schutzkolloid-Lösung zu erhalten. Dieser Schutzkolloid-Lösung wurde eine Vormischung aus 100 g Sonnenblumenöl und 4 g Vitamin D₃ unter starkem Rühren* zugesetzt. Dann wurde 60 g Covi-Ox T 70 EU (Gemisch aus D-α-Tocopherol, β - Tocopherol, *γ*-Tocopherol und *δ*-Tocopherol mit Sojaöl von BASF SE) zugegeben und eine Emulsion unter starkem Rühren* hergestellt. Die so hergestellte Emulsion wurde über eine Düse in einem Sprühturm sprühgekühlt, in dem Maisstärke (x50,3~5pm) bei 15° C fluidisiert wurde. Die noch feuchten Partikel wurden für 30 min bei 60° C Lufteintrittstemperatur im darunterliegenden Wirbelbett nachgetrocknet. Die so hergestellte Wirkstoffzubereitung wurde mit 3C bezeichnet. Die mittlere Partikelgröße in Dispersion war x50,3~6,4µm, die mittlere Partikelgröße des pulverförmigen Präparates war x50,3~280µm und die Restfeuchte des pulverförmigen Präparates 2,8%.

Stabilitätsprüfung für Lagerung von Wirkstoffzubereitungen mit Vitamin D₃ im Prämix Die Stabilität der so hergestellten Wirkstoffzubereitungen wurde in einem Stress-Prämixtest geprüft. Dafür wurden Prüfmuster von jeweils 100 mg der hergestellten Wirkstoffzubereitung und 4 g Prämixmischung in 50ml-Glasfläschen eingewogen. Die Prämixmischung bestand aus 60% Weizengrießkleie, 30% 50%-igem auf Kieselsäure geträgertem Cholinchlorid und 10% Spurenelementmischung. Die Spurenelementmischung bestand aus 46% FeSO₄x7H₂O, 38% CuSO₄x5H₂O, 12% ZnO und 4% MnO. Nach Zugabe aller Inhaltsstoffe wurden die Prüfmuster sorgfältig von Hand gemischt. Diese Prüfmuster wurden in einer Klimakammer bei 40° C und 70% für 4 Wochen gelagert. Vor Beginn der Lagerung und nach Abschluss der Lagerung wurde der Vitamin-D3-Gehalt der Prüfmuster bestimmt. Der Vitamin-D3-Gehalt wurde nach dem Methodenbuch der VDLUFA, Teil III, Kapitel 13.8.1 (VDLUFA Verlag Darmstadt, 1988) bestimmt. Aus dem Verhältnis der Vitamin-D₃-Gehalte nach und vor der Lagerung wurde die Retention berechnet.

Die Retentionswerte der Beispiele sind in der folgenden Tabelle zusammengefasst:

| Bezeichnung | Retention (%) |
|---|---|
| 3A | 35 |
| 3B | 12 |
| 3C | 17 |

Je höher die Retention desto besser die Stabilität der Wirkstoffzubereitung im Prämix. Vergleicht man die Stabilität der Wirkstoffzubereitung aus dem erfindungsgemäßen Bespiel mit den dazugehörigen nicht-erfindungsgemäßen Beispielen (3A mit 3B und 3C), so erkennt man deutlich die Stabilitätsverbesserung.

### Erfindungsgemäßes Beispiel 4A

Eine Stärke-Lösung wurde in einem beheizbaren Emulgierkessel bei 70° C hergestellt, indem 200 g modifizierte Stärke (PurityGum 2000 von Ingredion Germany GmbH) in 1500 g Wasser für 30 Minuten durch Quellen in Lösung gebracht wurden. Dieser Stärke-Lösung wurden 1000 g Lactose, 4 g Natriumcitrat, 4 g Natriumbenzoat und 20 g Emulgator (Lamemul K 2000 K von BASF SE) zugesetzt, um eine Schutzkolloid-Lösung zu erhalten. Dieser Schutzkolloid-Lösung wurden 80 g BHT unter starkem Rühren* zugesetzt. Dann wurden 350 g geschmolzenen Vitamin-A-Acetats zugegeben und eine Emulsion beim Durchgang durch einen Hochdruckhomogenisator** hergestellt. Die so hergestellte Emulsion wurde über eine Düse in einem Sprühturm bei 120° C Lufteintrittstemperatur und 90° C Luftaustrittstemperatur sprühgetrocknet. Die noch feuchten Partikel wurden für 45 min in einem nachgeschalteten Schaufeltrockner bei 60° C nachgetrocknet und vor der Nachtrocknung wurden 5 g Tricalciumphosphat (Phos4Pets 804 von Chemische Fabrik Budenheim KG) zugegeben. Die so hergestellte Wirkstoffzubereitung wurde mit 4A bezeichnet. Die mittlere Partikelgröße in Dispersion war x50,3~0,81pm, die mittlere Partikelgröße des pulverförmigen Präparates war x50,3~160pm und die Restfeuchte des pulverförmigen Präparates 3,0%.

### Nicht-erfindungsgemäßes Beispiel 4B

Eine Stärke-Lösung wurde in einem beheizbaren Emulgierkessel bei 70° C hergestellt, indem 200 g modifizierte Stärke (PurityGum 2000 von Ingredion Germany GmbH) in 1500 g Wasser für 30 Minuten durch Quellen in Lösung gebracht wurden. Dieser Stärke-Lösung wurden 1000 g Lactose, 4 g Natriumcitrat, 4 g Natriumbenzoat und 20 g Emulgator (Lamemul K 2000 K von BASF SE) zugesetzt, um eine Schutzkolloid-Lösung zu erhalten. 80 g BHT und 350 g geschmolzenen Vitamin-A-Acetat wurden separat gemischt und diese Mischung wurde der Schutzkolloid-Lösung unter starkem Rühren* zugesetzt. Nach der Zugabe wurde eine Emulsion beim Durchgang durch einen Hochdruckhomogenisator** hergestellt. Die so hergestellte Emulsion wurde über eine Düse in einem Sprühturm bei 120° C Lufteintrittstemperatur und 90° C Luftaustrittstemperatur sprühgetrocknet. Die noch feuchten Partikel wurden für 45 min in einem nachgeschalteten Schaufeltrockner bei 60° C nachgetrocknet und vor der Nachtrocknung wurden 5 g Tricalciumphosphat (Phos4Pets 804 von Chemische Fabrik Budenheim KG) zugegeben. Die so hergestellte Wirkstoffzubereitung wurde mit 4B bezeichnet. Die mittlere Partikelgröße in Dispersion war x50,3~0,68µm, die mittlere Partikelgröße des pulverförmigen Präparates war x50,3~180µm und die Restfeuchte des pulverförmigen Präparates 3,4%.

### Nicht-erfindungsgemäßes Beispiel 4C

Eine Stärke-Lösung wurde in einem beheizbaren Emulgierkessel bei 70° C hergestellt, indem 200 g modifizierte Stärke (PurityGum 2000 von Ingredion Germany GmbH) in 1500 g Wasser für 30 Minuten durch Quellen in Lösung gebracht wurden. Dieser Stärke-Lösung wurden 1000 g Lactose, 4 g Natriumcitrat, 4 g Natriumbenzoat und 20 g Emulgator (Lamemul K 2000 K von BASF SE) zugesetzt, um eine Schutzkolloid-Lösung zu erhalten. Dieser Schutzkolloid-Lösung wurden 350 g geschmolzenen Vitamin-A-Acetats unter starkem Rühren* zugesetzt. Dann wurden 80 g BHT zugegeben und eine Emulsion beim Durchgang durch einen Hochdruckhomogenisator** hergestellt. Die so hergestellte Emulsion wurde über eine Düse in einem Sprühturm bei 120° C Lufteintrittstemperatur und 90° C Luftaustrittstemperatur sprühgetrocknet. Die noch feuchten Partikel wurden für 45 min in einem nachgeschalteten Schaufeltrockner bei 60° C nachgetrocknet und vor der Nachtrocknung wurden 5 g Tricalciumphosphat (Phos4Pets 804 von Chemische Fabrik Budenheim KG) zugegeben. Die so hergestellte Wirkstoffzubereitung wurde mit 4C bezeichnet. Die mittlere Partikelgröße in Dispersion war x50,3~7,4µm, die mittlere Partikelgröße des pulverförmigen Präparates war x50,3~170pm und die Restfeuchte des pulverförmigen Präparates 3,2%.

### Stabilitätsprüfung für die Anwendung von Wirkstoffzubereitungen mit Vitamin A im Trinkwasser

Die Stabilität der so hergestellten Wirkstoffzubereitungen wurde für die Anwendung im Trinkwasser geprüft. Dafür wurden Prüfmuster von jeweils 100 mg der hergestellten Wirkstoffzubereitung in 10ml-Glasfläschen eingewogen und anschließend 8 ml Trinkwasser zugegeben. Diese Prüfmuster wurden bei 25° C für 24 Stunden gelagert. Unmittelbar mit Beginn der Lagerung (nach ca. 10 min) und nach Abschluss der Lagerung wurde der Vitamin-A-Gehalt der Prüfmuster bestimmt. Der Vitamin-A-Gehalt wurde nach Verordnung (EG) Nr. 152/2009, Anhang IV, Teil A bestimmt allerdings ohne Schritt 5.1. Aus dem Verhältnis der Vitamin-A-Gehalte nach der Lagerung und zu Beginn der Lagerung wurde die Retention berechnet.

Die Retentionswerte der Beispiele sind in der folgenden Tabelle zusammengefasst:

| Bezeichnung | Retention (%) |
|---|---|
| 4A | 81 |
| 4B | 78 |
| 4C | 54 |

Je höher die Retention desto besser die Stabilität der Wirkstoffzubereitung bei der Anwendung im Trinkwasser. Vergleicht man die Stabilität der Wirkstoffzubereitung aus den jeweiligen erfindungsgemäßen Bespielen mit den dazugehörigen nicht-erfindungsgemäßen Beispielen (4A mit 4B und 4C), so erkennt man deutlich die Stabilitätsverbesserung.
*) Energieeintrag beim Dispergieren (6500 rpm, ULTRA TURRAX UTL 2000 von IKA-Werke GmbH & Co KG) von 3×10⁷ J/m³
**) Energieeintrag beim Dispergieren (350 bar, Microfluidizer M-210 von Microfluidics Corp.) von 2×10⁸ J/m³

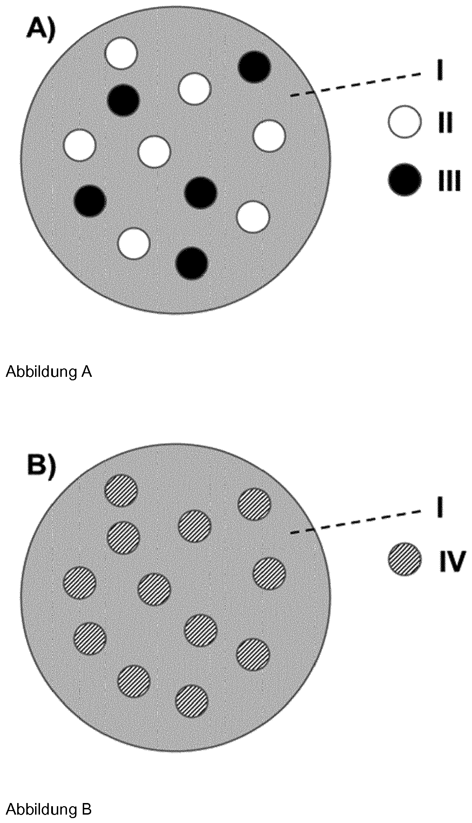

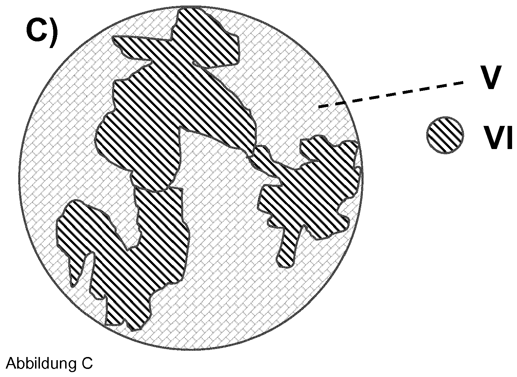

## Patentansprüche

1. Feste mindestens dreiphasige Wirkstoffzubereitungen, in denen zwei separate Phasen multipartikulär in eine kohärente wirkstoff- und antioxidationsfreie Phase eingebettet sind, wobei eine der eingebetteten Phasen antioxidationsfrei und mindestens einen schwer wasserlöslichen oxidationsempfindlichen Wirkstoff und die Zweite wirkstofffrei und mindestens ein schwer wasserlösliches Antioxidans enthält, wobei wirkstofffrei im Durchschnitt weniger als 1000ppm Wirkstoff und antioxidationsfrei im Durchschnitt weniger als 1000ppm Antioxidans und schwer wasserlöslich eine Löslichkeit von kleiner 0,1 Gew.-% in hochgereinigtem Wasser bei Standardbedingungen bedeutet.

## Claims

1. A solid, at least three-phase active compound preparation, in which two separate phases are embedded multiparticulately into a coherent, active compound-free and antioxidant-free phase, where one of the embedded phases is antioxidant-free and comprises at least one poorly water-soluble oxidation-sensitive active compound and the second is active compound-free and comprises at least one poorly water-soluble antioxidant, where active compound-free means on average less than 1000 ppm of active compound and antioxidant-free means on average less than 1000 ppm of antioxidant and poorly water-soluble means a solubility of less than 0.1 wt% in highly purified water under standard conditions.

## Revendications

1. Préparations solides de principes actifs à au moins trois phases, dans lesquelles deux phases distinctes sont incorporées de façon multiparticulaire dans une phase cohérente exempte de principe actif et d'antioxydation, l'une des phases incorporées étant exempte d'antioxydation et contenant au moins un principe actif difficilement soluble dans l'eau, sensible à l'oxydation et la deuxième étant exempte de principe actif et contenant au moins un antioxydant difficilement soluble dans l'eau, exempte de principe actif signifiant en moyenne moins de 1 000 ppm de principe actif et exempte d'antioxydation signifiant en moyenne moins de 1 000 ppm d'antioxydant et difficilement soluble dans l'eau signifiant une solubilité de moins de 0,1 % en poids dans de l'eau hautement purifiée, dans des conditions normalisées.
